# EUROPÄISCHE PATENTANMELDUNG

(11) **EP 1 302 779 A2**
(43) Veröffentlichungstag der Anmeldung: **16.04.2003**
(21) Anmeldenummer: 02102424.5
(22) Anmeldetag: 08.10.2002
(51) Int. Cl.: G01R 33/28, A61B 5/06

(54) **MR-Anordnung zur Lokalisierung eines medizinischen Instrumentes**

(30) Priorität: 10.10.2001 DE 10149955
(71) Anmelder: Philips Corporate Intellectual Property GmbH, 20099 Hamburg (DE); Koninklijke Philips Electronics N.V., 5621 BA Eindhoven (NL)
(72) Erfinder: Weiss, Steffen Philips Corp. Intel. PropertyGmbH, 52088, Aachen (DE)
(74) Vertreter: Volmer, Georg, Dipl.-Ing.

(57) **Zusammenfassung**

Die Erfindung betrifft eine MR-Anordnung mit einem medizinischen Instrument (3), einer daran angebrachten Hochfrequenzanordnung (17), in der wenigstens eine Mikrospule (1) und ein Kondensator (2) zu einem Resonanzkreis verschaltet sind, und mit einer optischen Signalleitung (4), über welche der Hochfrequenzanordnung ein Lichtsignal zugeführt wird. Zur einfachen und schnellen Bestimmung der Position des medizinischen Instrumentes (3) innerhalb des Untersuchungsbereiches eines MR-Gerätes schlägt die Erfindung vor, einen Modulator (20), zur Modulation des der Hochfrequenzanordnung zugeführten Lichtsignals, und einen opto-elektrischen Wandler (5) vorzusehen, welcher das modulierte Lichtsignal in ein elektrisches Signal umwandelt und der mit dem Resonanzkreis derart gekoppelt ist, daß der Resonanzkreis von dem elektrischen Signal des opto-elektrischen Wandlers (5) zur Abstrahlung eines Hochfrequenzfeldes angeregt wird.

## Beschreibung

Die Erfindung betrifft eine MR-Anordnung mit einem medizinischen Instrument, einer daran angebrachten Hochfrequenzanordnung, in der wenigstens eine Mikrospule und ein Kondensator zu einem Resonanzkreis verschaltet sind, und einer optischen Signalleitung, über welche der Hochfrequenzanordnung ein Lichtsignal zugeführt wird.

Die Erfindung betrifft ferner ein Verfahren zur Lokalisierung eines medizinischen Instrumentes im Untersuchungsvolumen eines MR-Gerätes, wobei das medizinische Instrument eine Hochfrequenzanordnung, in der wenigstes eine Mikrospule und ein Kondensator zu einem Resonanzkreis verschaltet sind, und eine optische Signalleitung aufweist, über welche der Hochfrequenzanordnung ein Lichtsignal zugeführt wird, und wobei aus dem von der Mikrospule in deren Umgebung erzeugten MR-Signal die Position des medizinischen Instrumentes ermittelt wird.

Außerdem betrifft die Erfindung ein medizinisches Instrument, insbesondere einen intravaskulären Katheter, einen Führungsdraht oder eine Biopsienadel, mit einer am distalen Ende angebrachten Hochfrequenzanordnung, in der wenigstens eine Mikrospule und ein Kondensator zu einem Resonanzkreis verschaltet sind, und mit einer optischen Signalleitung, über die der Hochfrequenzanordnung ein moduliertes Lichtsignal zugeführt wird, sowie ein MR-Gerät mit einem derartigen medizinischen Instrument.

In der Medizin spielt sowohl bei diagnostischen als auch bei therapeutischen Verfahren die Lokalisierung von interventionellen Instrumenten eine wichtige Rolle. Bei diesen Instrumenten kann es sich beispielsweise um intravaskuläre Katheter, Biopsienadeln, minimalinvasive chirurgische Instrumente oder ähnliches handeln. Eine wichtige Anwendung der interventionellen Radiologie ist die Angiographie, die dazu dient, die anatomischen Details des Gefäßsystems eines Patienten aufzuklären.

In letzter Zeit gewinnen auf Kernspintomographie (MR) basierende Angiographieverfahren an Bedeutung. Die Kernspinresonanz hat gegenüber der bisher üblichen Röntgendiagnostik den großen Vorteil der wesentlichen besseren Gewebeselektivität. Es sind MR-Techniken bekannt geworden, bei denen eine Mikrospule zur Detektion von Kernspinsignalen an einem interventionellen Instrument angeordnet ist. Von besonderem Interesse sind dabei MR-Verfahren zur Untersuchung von Blutgefäßen mittels intravaskulärer Katheter, an deren Spitze sich eine solche Mikrospule befindet.

Ein grundlegendes Problem bei derartigen MR-gestützten Angiographieverfahren beruht darauf, daß lange, entlang der gesamten Längserstreckung des intravaskulären Katheters verlaufende elektrische Verbindungsleitungen benötigt werden, um das hochfrequente MR-Signal von der an der Katheterspitze angebrachten Mikrospule zu der Empfangselektronik des verwendeten MR-Systems zu übertragen. In diesen Verbindungsdrähten kann es zu unerwünschten und gefährlichen Aufheizungserscheinungen kommen, welche durch die starke Hochfrequenzstrahlung im Untersuchungsbereich verursacht werden. Die Hochfrequenzfelder innerhalb des Untersuchungsbereiches können in den innerhalb des Katheters verlaufenden Kabeln stehende Wellen erzeugen, wodurch es zur resonanten Hochfrequenz-Aufheizung des Kabels kommt. Der Verwendung von intravaskulären Kathetern mit darin verlaufenden langen Kabeln treten die Sicherheit derartiger Anordnungen betreffende Bedenken entgegen. Die beschriebenen Phänomene sind nur schwer kalkulierbar, da die resonante Hochfrequenz-Aufheizung sowohl von den Feldverteilungen der Hochfrequenzfelder als auch von den geometrischen und elektrischen Eigenschaften der elektrischen Leitungen und deren dielektrischer Umgebung abhängt. In Experimenten sind plötzlich auftretende intensive Aufheizungen beobachtet worden, welche bei einem zu untersuchenden Patienten gegebenenfalls lebensbedrohliche Verletzungen verursachen können.

Die Gefahr der resonanten Hochfrequenz-Aufheizung wird gänzlich vermieden, wenn für die Übertragung der MR-Signale eine optische Übertragungstechnik verwendet wird.

Aus der US 6 236 205 B1 ist eine MR-Anordnung mit einem medizinischen Instrument sowie ein Verfahren zur Lokalisierung des medizinischen Instrumentes bekannt, wobei ein an dem medizinischen Instrument angebrachter Resonanzkreis über ein optisches Steuersignal, das dem Resonanzkreis über eine optische Faser zugeführt wird, beeinflußt wird. Bestandteil dieses Resonanzkreises ist wenigstens eine Mikrospule. Bei dem vorbekannten Verfahren wird zunächst in üblicher Weise im gesamten interessierenden Untersuchungsvolumen eine Hochfrequenz-Anregung durchgerührt. Durch die dabei in der Nähe des medizinischen Instrumentes erzeugte Kernmagnetisierung wird in der Mikrospule eine Spannung induziert. Der auf diese Weise angeregte Resonanzkreis strahlt daraufhin ein Hochfrequenzsignal ab, durch welches das MR-Signal in der Umgebung der Mikrospule verstärkt und verändert wird. Durch zeitliche Variation des optischen Steuersignals wird bei dem vorbekannten Verfahren der Resonanzkreis mittels einer optisch steuerbaren Impedanz abwechselnd einund ausgeschaltet. Das führt dazu, daß das von der Mikrospule abgestrahlte Hochfrequenzsignal entsprechend dem Steuersignal ebenfalls zeitlich variiert und somit vom Hintergrundsignal unterscheidbar wird. Dieses Unterscheidungskriterium wird dann zur Bestimmung der Position des medizinischen Instrumentes ausgenutzt.

Der Hauptnachteil des vorbekannten Verfahrens beruht darauf, daß dabei eine Magnetisierungsanregung im gesamten Untersuchungsvolumen erfolgen muß. Zur Positionsbestimmung wird ein Differenzsignal herangezogen, welches den Unterschied zwischen den detektierten MR-Signalen mit eingeschaltetem bzw. ausgeschaltetem Resonanzkreis wiedergibt. In diesem Differenzsignal konkurriert das schwache lokale Signal des Resonanzkreises mit dem starken Hintergrundsignal des gesamten Untersuchungs-volumens. Aus diesem Grund ist der Rauschabstand des für die Positionsbestimmung zur Verfügung stehenden Meßsignals vergleichsweise schlecht.

Ein weiterer Nachteil ist, daß das vorbekannte Verfahren in erheblichem Maße zusätzliche Meßzeit erfordert. Entweder müssen zunächst vollständige Bilder oder wiederholt Projektionen auf jede der drei Raumrichtungen generiert werden, um dann durch Differenzbildung die Position des medizinischen Instrumentes auffinden zu können. Die Wiederholung im Projektionsfalle ist nötig, um durch Mittelung den Rauschbestand des Messsignals zu erhöhen.

Außerdem ist nachteilig daß das vorbekannte Verfahren sehr anfällig für Bewegungsartefakte ist. Wird die Position des medizinischen Instrumentes im Verlauf der Messung geändert, so führt dies zu Fehlern in den Bilddaten, was eine zuverlässige Lokalisierung stark erschwert.

Es ist Aufgabe der vorliegenden Erfindung, eine verbesserte MR-Anordnung bereitzustellen, wobei insbesondere die beschriebenen Nachteile vermieden werden sollen.

Diese Aufgabe wird ausgehend von einer MR-Anordnung der eingangs genannten Art gelöst durch einen Modulator, zur Modulation des der Hochfrequenzanordnung zugeführten Lichtsignals, und einen opto-elektrischen Wandler, der das modulierte Lichtsignal in ein elektrisches Signal umwandelt und der mit dem Resonanzkreis derart gekoppelt ist, daß der Resonanzkreis von dem elektrischen Signal des opto-elektrischen Wandlers zur Abstrahlung eines Hochfrequenzfeldes angeregt wird.

Bei der Anordnung gemäß der vorliegenden Erfindung wird das modulierte Lichtsignal mittels des opto-elektrischen Wandlers in ein elektrisches Signal umgewandelt, wodurch der Resonanzkreis zur Abstrahlung eines Hochfrequenzsignals angeregt wird Durch das abgestrahlte Hochfrequenzsignal kommt es dann in der unmittelbaren räumlichen Umgebung der Mikrospule zur Erzeugung von Kernmagnetisierung. Diese lokale Kernmagnetisierung kann als MR-Signal mittels der externen Empfangsspulen des MR-Gerätes sehr leicht detektiert und ohne aufwendige Verarbeitung, im einfachsten Fall durch eine bloße Frequenzanalyse, zur Positionsbestimmung herangezogen werden.

Anders als bei der vorbekannten Anordnung kommt bei der Anordnung gemäß der vorliegenden Erfindung anstelle der optisch gesteuerten Impedanz der opto-elektrische Wandler zum Einsatz, welcher gewissermaßen als Stromquelle für den Resonanzkreis dient und so die aktive Erzeugung lokaler Kernmagnetisierung ermöglicht. Hierfür muß das Lichtsignal mit der Resonanzfrequenz des verwendeten MR-Gerätes moduliert werden.

Gegenüber dem vorbekannten Verfahren erfolgt die Positionsbestimmung mit der erfindungsgemäßen Anordnung durch eine lokale und nicht durch eine globale Hochfrequenzanregung. Daraus resultiert der Vorteil, daß das oben beschriebene aufwendige und fehleranfällige Differenzverfahren zur Positionsbestimmung gänzlich entfällt. Mit der erfindungsgemäßen Anordnung erfordert die Positionsbestimmung vorteilhafterweise nur ein Minimum an Meßzeit. Bewegungsartefakte stellen dementsprechend kein Problem dar.

Gemäß Anspruch 2 kann der opto-elektrische Wandler der erfindungsgemäßen Anordnung zweckmäßigerweise eine einfache Photodiode sein. Die Anregung des Resonanzkreises erfolgt dann durch den Photostrom, der von dem modulierten Lichtsignal in der Photodiode erzeugt wird. Geeignete Photodioden sind vorteilhafterweise als kompakte Bauelemente zu geringen Kosten im Handel erhältlich.

Eine besonders einfache Anordnung ergibt sich gemäß Anspruch 3 dadurch, daß in dem Resonanzkreis die Mikrospule, der Kondensator und die Photodiode parallel geschaltet sind. Es ergibt sich damit ein Parallelresonanzkreis aus Mikrospule und Kondensator, wobei der Photostrom der ebenfalls parallel geschalteten Photodiode direkt in den Parallelresonanzkreis eingespeist wird.

Die erfindungsgemäße Anordnung kann für ein Verfahren zur Lokalisierung eines medizinischen Instrumentes gemäß Anspruch 4 zum Einsatz kommen. Zur Erzeugung der lokalen Kernmagnetisierung bzw. des detektierbaren MR-Signals ist es zweckmäßig, gemäß Anspruch 5 die Einspeisung des modulierten Lichtsignals pulsweise auszuführen. Das Lichtsignal wird als Lichtpuls eingespeist, wobei das Lichtsignal gleichzeitig mit der Resonanzfrequenz des MR-Gerätes moduliert ist. Aus diesem Lichtpuls erzeugt der opto-elektrische Wandler einen Hochfrequenzpuls, der den üblichen zur Erzeugung von Kernmagnetisierung dienenden Hochfrequenzpulsen entspricht. Die Länge und Form des Pulses kann so angepaßt werden, daß eine optimale Anregung erzielt wird. Die Wahl der entsprechenden Parameter hängt von der gewünschten räumlichen Auflösung bei der Positionsbestimmung sowie von der zur Detektion benötigten Amplitude des MR-Signals ab. Alternativ wird das Licht gemäß Anspruch 6 kontinuierlich eingespeist, wobei lediglich die Modulation mit der Resonanzfrequenz des MR-Gerätes pulsweise erfolgt. Dies kann dann vorteilhaft sein, wenn als opto-elektrischer Wandler beispielsweise eine Photodiode verwendet wird, deren Impedanz von der Lichteinstrahlung abhängt. Die Impedanz der Photodiode beeinflußt direkt die Resonanzeigenschaften des Resonanzkreises. Es ist auf einfache Weise möglich, den Resonanzkreis so abzustimmen, daß die Resonanzbedingung erfüllt ist, wenn die Photodiode beleuchtet wird, wenn also ein Photostrom fließt. Dies hat den weiteren Vorteil, daß der Resonanzkreis auch während der Detektionsphase in Resonanz ist, was dazu führt, daß das MR-Signal aus der Umgebung des medizinischen Instrumentes zusätzlich verstärkt wird.

Ein gemäß der Erfindung verwendbares medizinisches Instrument ist Gegenstand der Ansprüche 7 bis 9. Nach Anspruch 9 ist es zweckmäßig, einen zusätzlichen Sperrkondensator in der Parallelschaltung aus Mikrospule, Kondensator und Photodiode vorzusehen. Durch den Sperrkondensator wird die Gleichstromkomponente der Photodiode blockiert und von dem Resonanzkreis ferngehalten. Ohne den Sperrkondensator besteht die Gefahr, daß sich die Mikrospule aufheizt. Der Sperrkondensator bietet weiterhin den Vorteil, daß durch Variation des Gleichanteils im Lichtsignal der Arbeitspunkt der Photodiode und damit auch ihre Impedanz geändert werden kann. Dies läßt sich zur Feinabstimmung des Resonanzkreises verwenden, die durch Änderungen der Resonanzfrequenz des Schwingkreises hervorgerufen durch Änderungen der dielektrischen Umgebung, Temperaturdrifts oder geometrische Deformierungen der Mikrospule nötig werden können.

Gemäß Anspruch 10 kann ein herkömmliches MR-Gerät problemlos mit einer erfindungsgemäßen MR-Anordnung ausgestattet werden.

Im folgenden werden Ausführungsbeispiele der Erfindung anhand der Zeichnungen erläutert.

Es zeigen:
Fig. 1 Intravaskulärer Katheter gemäß der Erfindung;
Fig. 2 MR-Gerät gemäß der Erfindung.

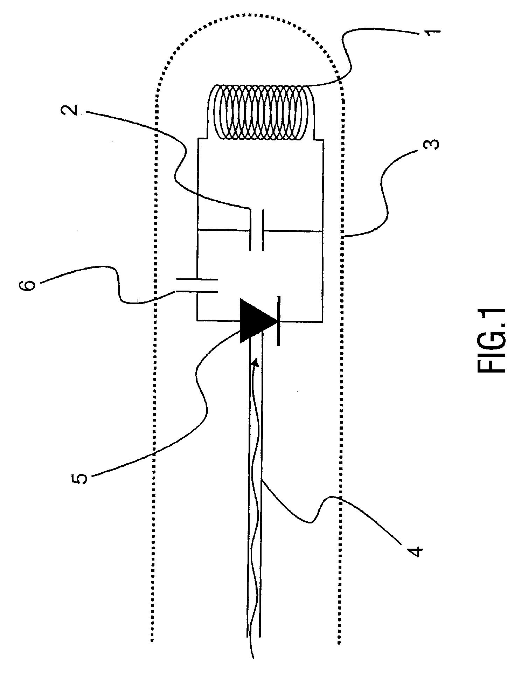

Anhand der Figur 1 wird deutlich, daß es problemlos möglich ist, gemäß der Erfindung eine Hochfrequenzanordnung, in der eine Mikrospule 1 und ein Kondensator 2 zu einem Resonanzkreis verschaltet sind, in die Spitze eines intravaskulären Katheters 3 zu integrieren. Der Hochfrequenzanordnung wird ein Lichtsignal über eine Signalleitung 4 zugeführt. Bei der Signalleitung 4 kann es sich beispielsweise um eine herkömmliche Glasfaser handeln. Bei dem in der Figur 1 gezeigten Ausführungsbeispiel kommt als opto-elektrischer Wandler eine Photodiode 5 zum Einsatz. Durch das modulierte Lichtsignal, das der Photodiode 5 über die optische Faser 4 zugeführt wird, wird in der Photodiode 5 ein Photostrom erzeugt. Das Lichtsignal ist mit der Frequenz des Resonanzkreises moduliert, so daß dieser durch den resultierenden hochfrequenten Photostrom resonant angeregt wird. Aufgrund des dabei in dem Resonanzkreis fließenden Wechselstroms strahlt die Mikrospule 1 ein Hochfrequenzfeld in die Umgebung der Spitze des Katheters 3 ab. Bestandteil der in der Figur 1 gezeigten Anordnung ist des weiteren ein Sperrkondensator 6, durch den die Gleichstromkomponente des in der Photodiode 5 erzeugten Photostroms von dem Resonanzkreis abgehalten wird. Die Impedanzen der Photodiode 5 und des Sperrkondensators 6 beeinflussen das Resonanzverhalten der Hochfrequenzanordnung. Es ist zweckmäßig, den Resonanzkreis so abzustimmen, daß die Resonanzfrequenz den gewünschten Wert hat, wenn die Photodiode im aktiven, also beleuchteten Zustand ist. Durch Variation der Intensität des eingespeisten Lichtsignals besteht dann vorteilhafterweise die Möglichkeit, eine Feinabstimmung des Resonanzkreises in gewissen Grenzen durchzuführen, um beispielsweise Änderungen in der dielektrischen Umgebung des Kreises, Drift durch Temperatureinflüsse oder geometrische Deformationen der Mikrospule zu kompensieren.

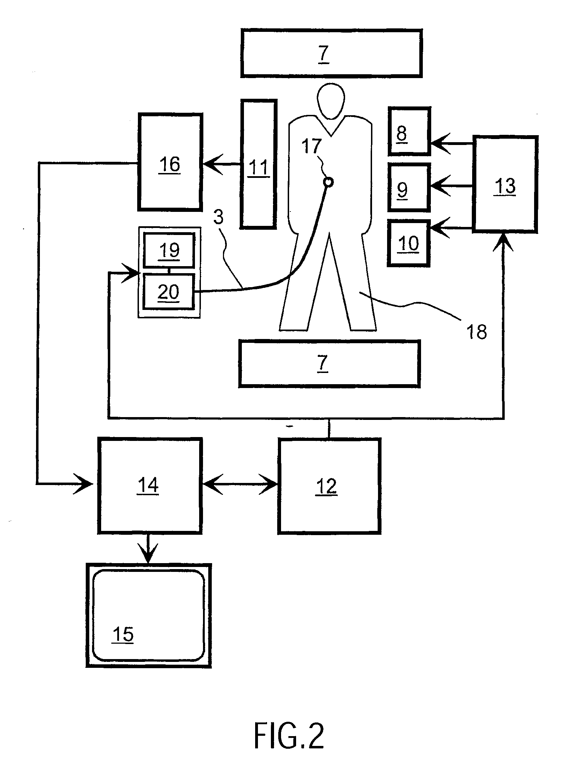

Die Figur 2 zeigt ein MR-Gerät, das mit einer erfindungsgemäßen Anordnung ausgestattet ist, als Blockdiagramm. Das System besteht aus einer Hauptfeldspule 7 zur Erzeugung eines statischen homogenen Magnetfeldes, Gradienten-Spulen 8, 9 und 10, zur Erzeugung von Magnetfeldgradienten in X-, Y- und Z-Richtung, und aus einer Empfangsspule 11, zur Detektion von MR-Signalen im Untersuchungsvolumen des MR-Gerätes. Mittels einer Steuerungseinheit 12, die über einen Gradientenverstärker 13 mit den Gradientenspulen 8,9 und 10 in Verbindung steht, wird der zeitliche Verlauf der Magnetfeldgradienten gesteuert. Zum System gehört ferner als Rekonstruktionseinheit ein Mikrocomputer 14 sowie eine Visualisierungseinheit 15, etwa in Form eines Grafikmonitors. Die Empfangsspule 11 ist über eine Empfangseinheit 16 mit der Rekonstruktionseinheit 14 verbunden, welche die detektierten MR-Signale verarbeitet und in geeigneter Weise mittels der Visualisierungseinheit 15 darstellt. Die Empfangseinheit 16 arbeitet in der üblichen Weise und demoduliert das MR-Signal, um es möglichst schmalbandig in ein rauscharmes niederfrequentes Meßsignal umzuwandeln.

Die Hochfrequenzanordnung des Katheters 3 ist in der Figur 2 als Ganzes mit der Bezugsziffer 17 bezeichnet. Der Katheter 3 ist in einen Patienten 18 eingeführt. Die Hochfrequenzanordnung 17 steht über die im Katheter 3 verlaufende optische Faser 4 mit einer Lichtquelle 19 und einem Modulator 20, zur Modulation des Lichtes der Lichtquelle 19, in Verbindung. Die Lichtquelle 19 und der Modulator 20 werden von der Steuereinheit 12 angesteuert, welche den zeitlichen Verlauf des eingespeisten Lichtsignals kontrolliert. Bei dem Modulator 20 kann es sich beispielsweise um eine Pockels-Zelle handeln, bei der ein Kristall aus elektro-optischem Material zwischen zwei gekreuzten Polarisatoren angeordnet ist. Die in die optische Signalleitung 4 des Katheters 3 eingespeiste Lichtintensität ist dabei proportional zu einer an den Modulator 20 angelegten hochfrequenten Wechselspannung, deren zeitlicher Verlauf von der Steuereinheit 12 vorgegeben wird.

Während der Untersuchung befindet sich der Katheter 3 in einem Blutgefäß im Inneren des Patienten 18. In dem die Katheterspitze unmittelbar umgebenden Gewebe wird durch das von der Mikrospule abgestrahlte Hochfrequenzfeld lokal Kernmagnetisierung erzeugt, welche als MR-Signal mittels der externen Empfangspule 11 des MR-Gerätes detektierbar ist. Durch Überlagerung des von der Hauptfeldspule 7 erzeugten Magnetfeldes mit Magnetfeldgradienten in geeigneter Weise wird die gewünschte Ortsinformation in der Frequenz des aus der Umgebung der Katheterspitze stammenden MR-Signals kodiert. Die Position der Spitze des Katheters 3 innerhalb des Patienten 18 ergibt sich dann einfach durch Bestimmung der Frequenz des detektierten MR-Signals. Die Frequenzanalyse des MR-Signals erfolgt mittels der Rekonstruktionseinheit 14, welche dann die Position der Katheterspitze auf dem Monitor 15 anzeigt.

## Patentansprüche

1. MR-Anordnung mit einem medizinischen Instrument (3), einer daran angebrachten Hochfrequenzanordnung (17), in der wenigstens eine Mikrospule (1) und ein Kondensator (2) zu einem Resonanzkreis verschaltet sind, und mit einer optischen Signalleitung (4), über welche der Hochfrequenzanordnung ein Lichtsignal zugeführt wird, **gekennzeichnet durch** einen Modulator (20), zur Modulation des der Hochfrequenzanordnung zugeführten Lichtsignals, und einen opto-elektrischen Wandler (5), der das modulierte Lichtsignal in ein elektrisches Signal umwandelt und der mit dem Resonanzkreis derart gekoppelt ist, daß der Resonanzkreis von dem elektrischen Signal des opto-dektrischen Wandlers (5) zur Abstrahlung eines Hochfrequenzfeldes angeregt wird.

2. MR-Anordnung nach Anspruch 1,
**dadurch gekennzeichnet,**
**daß** der opto-elektrische Wandler (5) eine Photodiode ist.

3. MR-Anordnung nach Anspruch 2,
**dadurch gekennzeichnet,**
**daß** in dem Resonanzkreis die Mikrospule (1), der Kondensator (2) und die Photodiode (5) parallel geschaltet sind.

4. Verfahren zur Lokalisierung eines medizinischen Instrumentes (3) im Untersuchungsvolumen eines MR-Gerätes, wobei das medizinische Instrument (3) eine Hochfrequenzanordnung (17), in der wenigstes eine Mikrospule (1) und ein Kondensator (2) zu einem Resonanzkreis verschaltet sind, und eine optische Signalleitung (4) aufweist, über welche der Hochfrequenzanordnung ein Lichtsignal zugeführt wird, und wobei aus dem von der Mikrospule (1) in deren Umgebung erzeugten MR-Signal die Position des medizinischen Instrumentes (3) ermittelt wird, **dadurch gekennzeichnet, daß** das MR-Signal erzeugt wird, indem in die optische Signalleitung (4) ein mit der Resonanzfrequenz des MR-Gerätes moduliertes Lichtsignal eingespeist wird, welches mittels eines mit dem Resonanzkreis gekoppelten opto-elektrischen Wandlers (5) in ein elektrisches Signal umgewandelt wird, durch welches der Resonanzkreis zur Abstrahlung eines Hochfrequenzfeldes mit der Resonanzfrequenz des MR-Gerätes angeregt wird.

5. Verfahren nach Anspruch 4,
**dadurch gekennzeichnet,**
**daß** die Einspeisung des modulierten Lichtsignals in die optische Signalleitung (4) pulsweise erfolgt, wobei das in der Umgebung der Mikrospule (1) erzeugte MR-Signal jeweils im Anschluß an einen Lichtpuls detektiert und dann zur Bestimmung der Position ausgewertet wird.

6. Verfahren nach Anspruch 4,
**dadurch gekennzeichnet,**
**daß** das in die optische Signalleitung (4) eingespeiste Lichtsignal pulsweise mit der Resonanzfrequenz des MR-Gerätes moduliert wird, wobei das in der Umgebung der Mikrospule (1) erzeugte MR-Signal zur Positionsbestimmung jeweils in denjenigen Zeitintervallen detektiert wird, in denen das Lichtsignal unmoduliert ist.

7. Medizinisches Instrument, insbesondere intravaskulärer Katheter, Führungsdraht oder Biopsienadel, mit einer am distalen Ende angebrachten Hochfrequenzanordnung (17), in der wenigstens eine Mikrospule (1) und ein Kondensator (2) zu einem Resonanzkreis verschaltet sind, und mit einer optischen Signalleitung (4), über die der Hochfrequenzanordnung (17) ein moduliertes Lichtsignal zugeführt wird, **dadurch gekennzeichnet, daß** der Resonanzkreis mit einer Photodiode (5), in der durch das modulierte Lichtsignal ein Photostrom erzeugt wird, derart gekoppelt ist, daß der Resonanzkreis von dem Fhotostrom zur Abstrahlung eines Hochfrequenzfeldes angeregt wird.

8. Medizinisches Instrument nach Anspruch 7,
**dadurch gekennzeichnet,**
**daß** in dem Resonanzkreis die Mikrospule (1), der Kondensator (2) und die Photodiode (5) parallel geschaltet sind.

9. Medizinisches Instrument nach Anspruch 8,
**dadurch gekennzeichnet,**
**daß** ein zusätzlicher Sperrkondensator (6) vorgesehen ist, welcher mit der Photodiode (5) in Serie geschaltet ist.

10. MR-Gerät mit wenigstens einer Hauptfeldspule (7), einer Anzahl von Gradientenspulen (8,9,10), wenigstens einer Steuerungseinheit (12), wenigstens einer Empfangsspule (11), die an eine Empfangseinheit (16) angeschlossen ist, einer Datenverarbeitungseinheit (14) und einem medizinischen Instrument (3), das eine Hochfrequenzanordnung (17) aufweist, in welcher wenigstens eine Mikrospule (1) und ein Kondensator (2) zu einem Resonanzkreis verschaltet sind, wobei der Hochfrequenzanordnung (17) mittels einer optischen Signalleitung (4) ein Lichtsignal zugeführt wird, **dadurch gekennzeichnet, daß** das Licht einer Lichtquelle (19) vor der Einspeisung in die optische Signalleitung (4) mittels eines Modulators (20) moduliert wird und daß das modulierte Lichtsignal mittels eines opto-elektrischen Wandlers (5) in ein elektrisches Signal umgewandelt wird, wobei der opto-elektrische Wandler (5) derart mit dem Resonanzkreis gekoppelt ist, daß der Resonanzkreis von dem elektrischen Signal des opto-dektrischen Wandlers (5) zur Abstrahlung eines Hochfrequenzfeldes angeregt wird.
